# EUROPÄISCHE PATENTSCHRIFT

(11) **EP 0 894 507 B1**
(45) Veröffentlichungstag und Bekanntmachung des Hinweises auf die Patenterteilung: **26.09.2001**
(21) Anmeldenummer: 98113879.5
(22) Anmeldetag: 24.07.1998
(51) Int. Cl.: A61M 25/10

(54) **Ballonkatheter**
Balloon Catheter
Cathéther à ballonnet

(30) Priorität: 31.07.1997 DE 19732965
(43) Veröffentlichungstag der Anmeldung: 03.02.1999
(73) Patentinhaber: WILLY RÜSCH AG, D-71394 Kernen-Rommelshausen (DE)
(72) Erfinder: Knörig, Joachim-Michael, Dr., 12589 Berlin (DE)
(74) Vertreter: KOHLER SCHMID + PARTNER

(56) Entgegenhaltungen:
- EP-A- 0 204 218
- EP-A- 0 218 275
- EP-A- 0 490 459
- US-A- 5 423 745
- US-A- 5 620 418

## Beschreibung

Die Erfindung geht aus von einem Ballonkatheter zur Anwendung in der Bypasschirurgie mit einem am Katheterschaft ausgebildeten aufblasbaren Ballon zur Blockung des Lumens der Aorta, wobei ein Führungslumen im Katheterschaft und der Ballon in einem Spitzenbereich des Katheterschaftes ausgebildet sind, mit einem einen Kardioplegiekanal bildenden ersten Lumen zur Infusion einer Kardioplegielösung über mindestens ein erstes Auge und mit einem einen Ventkanal bildenden zweiten Lumen zur Entlastung des Herzens und/oder zum Absaugen von Blut.

EP 0218275 A1 offenbart einen Ballonkatheter zur Anwendung in der Bypasschirurgie mit einem am Katheterschaft ausgebildeten aufblasbaren Ballon zur Blockung des Lumens der Aorta, wobei ein Führungslumen im Katheterschaft und der Ballon in einem Spitzenbereich des Katheterschaftes ausgebildet sind, mit einem einen Kardioplegiekanal bildenden ersten Lumen zus Infusion einer Kardioplegielösung über mindestens ein erstes Auge und mit einem, einen Ventkanal bildenden zweiten Lumen zur Entlastung des Herzens und/oder Absaugen von Blut, und wobei die Außenoberfläche des Ballons eine mit Rippen versehene, struktruierte Oberfläche darstellt.

EP 0204218 A1 zeigt eine strukturierte Oberfläche des Ballons.

Durch den Ballonkatheter ist eine gleichzeitige Herzentlastung und Kardioplegie möglich.

Weiterhin sind aus der Hezrchirurgie Blockungskatheter bekannt, die über die Leiste in den Aortenbogen vorgeschoben werden. Die Plazierung eines derartigen Blockungskatheters ist technisch schwierig, mit Komplikationen behaftet und zeitaufwendig. Außerdem ist eine Herzentlastung des stillgelegten und kardioplegierten Herzens nicht möglich.

Aufgabe der Erfindung ist es, einen Ballonkatheter zu schaffen, der die fluiddichte und lagestabile Occlusion der Aorta zuverlässig gewährleistet, das occludierte Hohlorgan im Bereich der Blockung schont, einen kurzen und vereinfachten Implantationsweg eröffnet und gleichzeitig die Herzentlastung und Kardioplegie ermöglicht.

Diese Aufgabe wird erfindungsgemäß durch einen Ballonkatheter gelöst, bei dem das Führungslumen am patientenseitigen Ende geschlossen ist und in einer Katheterspitze ausläuft, dass der Ballon eine strukturierte und/oder zerklüftete Außenoberfläche aufweist, dass die Lumen durch eine erste Katheterummantelung, die am patientenabgewandten Ende des Ballons endet, und eine zweite Katheterummantelung ausgebildet sind, die auf der ersten Katheterummantelung angeordnet und axial beabstandet von dem ersten Auge endet, wobei die zweite Katheterummantelung mindestens ein zweites Auge aufweist.

Der erfindungsgemäße Ballonkatheter hat damit den wesentlichen Vorteil, daß sich der Ballon an die Gefäßintima der Aorta unverrückbar anpressen läßt. Die strukturierte Außenoberfläche des gummielastischen Ballons kann sich verbessert an Gefäßinnenoberflächen unter erhöhten Reibungswerten anlegen, ohne daß die Gefäßstruktur in dem occludierten Bereich verletzt wird. Die Außenoberfläche des gummielastischen Ballons weist Berge und Täler auf, die sich an Unebenheiten an Gefäßinnenwandungen anpassen und sich bei aufgeblasenem Ballon mit den Unebenheiten oder vorgegebenen Gefäßstrukturen verbinden, so daß ein plazierter und aufgeblasener Ballon erstens seine Lage auch unter Druck nicht verändert und zweitens eine fluiddichte Absperrung eines Hohlorgans sicher gewährleistet ist.

Ist die strukturierte und/oder zerklüftete Außenoberfläche des Ballons auch visuell erkennbar, so ist gesichert, daß der eingesetzte Katheter auch einen funktionsgerechten Ballon aufweist. Ist der Ballon zusätzlich noch aus Latex gefertigt, so können die überragenden Materialeigenschaften - extreme Dehnfähigkeit, gutes Rückstellvermögen - für die Blockung eines Hohlorgans vorteilhaft eingesetzt werden.

Der Latex-Ballon kann über ein Tauchverfahren hergestellt werden, und in einem sich unmittelbar daran anschließenden Verfahrensschritt wird der auf einer gewünschten Ballonform befindliche Latex-Milchfilm in eine vorbestimmte Lösung getaucht, die die Außenoberfläche des Latex-Ballons unregelmäßig strukturiert oder zerklüftet, ohne die Dehnfähigkeit des Latexmaterials für den hier vorgesehenen Anwendungsbereich nachteilig zu beeinflussen. Die Strukturierung der Außenoberfläche des Ballons könnte aber auch mechanisch oder mechanisch und thermisch erfolgen. Die vorgesehene Oberflächenstrukturierung über geeignete Lösungen hat im Herstellungsprozeß des erfindungsgemäßen Ballons weiterhin den Vorteil, daß dieser Verfahrensschritt einfach und zeitgünstig durchzuführen ist.

In weiterer Ausgestaltung der Erfindung ist der Latex-Ballon in einer eigenstabilen Grundform ausgebildet, die in einem ersten und in einem zweiten Endbereich an Schaftabschnitte eines Katheters angepaßt ist.

Dies hat den Vorteil, daß die Grundgröße des hergestellten Ballons definiert klein gehalten werden kann und geeignete Endabschnitte des Ballons, beispielsweise gegenüber einem Schaftabschnitt eines Katheters auf Untermaß gefertigt, gewährleisten, daß sich diese Endbereiche beim Aufbringen auf einen Schaftabschnitt faltenfrei an dessen Außenoberfläche anschmiegen, sich mit dieser verkleben und/oder sich über Fäden am Katheterschaft fluiddicht und druckdicht befestigen lassen.

Gemäss der Erfindung weist der Ballonkatheter im Katheterschaft ein Führungslumen auf, das am patientenseitigen Ende geschlossen ist und in einer Katheterspitze endet, die bevorzugt verdickt ausgebildet ist. Im Spitzenbereich des Ballonkatheters ist der Ballon ausgebildet und dort ortsfest angebracht. Der Ballonkatheter weist zusätzlich eine erste Katheterschaftummantelung auf, die am patientenabgewandten Ende des Ballons endet und mit mindestens einem ersten Auge versehen ist. Das erste Auge steht mit einem ersten Lumen in Verbindung und über das erste Auge ist das erste Lumen mit der Umgebung verbunden. Zusätzlich ist an dem Ballonkatheter noch eine zweite Katheterummantelung vorgesehen, die auf der ersten Katheterummantelung angeordnet ist und beabstandet von dem ersten Auge endet, wobei die zweite Katheterummantelung mindestens ein zweites Auge aufweist, das ein zweites Lumen mit der Umgebung verbindet.

Durch die erfindungsgemäße Gestaltung des Ballonkatheters ergeben sich im wesentlichen drei Einsatzgebiete, die zum Teil neue Praktiken in der Herzchirurgie ermöglichen. Der Ballonkatheter mit dem an der Außenoberfläche strukturierten Ballon kann bei operativen Eingriffen eingesetzt werden,
1. die durch die Verkalkung der Aorta eine Querklemmung nicht gestatten;
2. zur Erstellung von LIMA/RIMA-Anastomosen, die unter Anwendung der MIC-Technik vorgenommen werden; und
3. die unter Anwendung der MIC-Technik zur Erstellung von Venenanastomosen zwischen Koronargefäß und Aorta descendens dienen.

Die Abkürzung "LIMA" bzw. "RIMA" steht für die Bezeichnungen linke Arteria mammaria interna bzw. rechte Arteria mammaria interna (neue Nomenklatur: Arteria thoracica interna links bzw. rechts).

Weist der Ballonkatheter die erfindungsgemäßen Merkmale auf, so kann er als transventrikulärer Blockungsballon der Aorta mit Kardioplegie- und Ventkanal zur Anwendung in der Bypasschirurgie eingesetzt werden. Mit dem erfindungsgemäßen Ballonkatheter wird eine Aortenocclusion und Kardioplegie ohne Klemmung der arteriosklerotischen Aorta ermöglicht. Eine Aortenklemmung (Stand der Technik) kann zu einer Beschädigung der Aortenwand und zur Abschwemmung von Kalkpartikein ins Gehirn mit embolischen Ausfällen führen.

Nachdem die Anzahl der Patienten im Endzustand einer Arteriosklerose zunimmt, und immer häufiger auch Patienten von Reoperationen betroffen sind, wird durch die Verwendung des erfindungsgemäßen Ballonkatheters der chirurgische Eingriff am Herzen eines Patienten vereinfacht. Der erfindungsgemäße Ballonkatheter weist einen Führungskanal für die Nutzung eines Mandrin auf. Über den aufblasbaren Latex-Ballon mit zerklüfteter Außenoberfläche läßt sich die Aorta zuverlässig blocken und über ein erstes Lumen kann Kardioplegielösung zwischen Ballon und Aortenklappe in die Aorta und Herzkranzgefäße einströmen. Über ein zweites Lumen wird das kardioplegische Herz entlastet und das vom zweiten Lumen aufgenommene Blut zur Herz-Lungenmaschine transportiert. Der erfindungsgemäße weitergebildete Ballonkatheter kann zur sicheren Blockung der Aorta ascendens bei Bypassoperationen herangezogen werden, auch dann, wenn die Aorta ascendens in diesem Bereich starke Verkalkungen aufweist. Eine aus dem Stand der Technik bekannte Querklemmung der Aorta könnte zu einer Aortenzerstörung führen. Der erfindungsgemäß weitergebildete Ballonkatheter könnte aber auch bei Operationen im Rahmen der bisher üblichen minimalinvasiven Koronarchirurgie eingesetzt werden sowie innerhalb der minimalinvasiven Koronarchirurgie in einer neuen Technik, die es gestattet, auch Venenbypässe zu legen.

Bei Bypassoperationen stellen Verkalkungen in der Aorta ascendens große medizinische Probleme dar. Im Rahmen eines operativen Eingriffs muß die Aorta ascendens quer geklemmt werden, so daß die Gefahr einer Beschädigung der verkalkten Aorta sehr groß ist. Tritt in diesem Zusammenhang eine Gefäßbeschädigung bzw. eine Gefäßzerstörung auf, so sind die sich daraus ergebenden medizinischen Probleme nur sehr schwer beherrschbar und können nur durch eine lang andauernde Operation behoben werden. Zusätzlich kann es bei der Abklemmung der Aorta ascendens zu einem Abschwemmen von Kalkpartikeln ins Gehirn kommen. Dies kann zu dem klinischen Bild eines Hirninfarktes mit motorischen oder sensiblen Ausfällen von Hirnleistungen oder zum Koma führen.

Bisher wurden diese Komplikationen durch die Durchführung der Operation in tiefer Hypothermie umgangen. Die Patienten werden mit der Herz-Lungenmaschine bis zu Temperaturen von ca. 17 °C Körperkerntemperatur abgekühlt. Bei dieser Temperatur wird der Zellstoffwechsel auf ein Mindestmaß herabgesetzt. Zu diesem Zeitpunkt wird die Herz-Lungenmaschine angehalten und der Kreislauf unterbrochen. Während dieses Zeitraums werden die peripheren Koronaranastomosen genäht. Die Dauer des Kreislaufstillstandes ist auf ca. 60 Minuten begrenzt. Im Anschluß erfolgt die Aufnahme des Kreislaufes und die Wiedererwärmung des Patienten. Es ist nachvollziehbar, daß dieses Vorgehen gerade bei alten und multimorbiden Patienten zu schweren postoperativen Störungen führen kann. Am Gehirn dieser Patienten können sich schwerwiegende Folgen zeigen. Die Patienten zeigen Durchgangssyndrome mit Verwirrtheitszuständen, die oft eine lange und komplizierte intensivmedizinische Betreuung erforderlich machen. Weitere Organausfälle sind möglich. Neben diesen medizinischen Folgen sind auch finanzielle Belastungen vorhanden. So nimmt die Kühlung und Wiedererwärmung einen langen Operationszeitraum in Anspruch. Die lange postoperative Betreuung und notwendige Medikation ist äußerst kostenintensiv. Diese vorgenannten Probleme können mit dem Einsatz des erfindungsgemäß weiterentwickelten Ballonkatheters umgangen werden und zeigen einen neuen alternativen Weg der Bypasschirurgie auf.

In bevorzugter Ausgestaltung des erfindungsgemäßen Ballonkatheters ist längs des Katheterschaftes eine visuell erkennbare Gradierung aufgebracht bzw. in das Kathetermaterial eingearbeitet. Dies hat den Vorteil, daß der operierende Arzt die Einschubtiefe des Ballons im Herz am Katheterschaft ablesen kann.

Neben der erfindungsgemäßen Vorrichtung zur Vereinfachung von Bypassoperationen wird ein Verfahren zur Anwendung des Ballonkatheters vorgestellt, bei dem der Ballonkatheter über eine Ventrikulomie von außen in das Herz eines an einer Herz-Lungenmaschine angeschlossenen Patienten über die Spitze des linken Ventrikels eingeführt wird, bei dem der Ballonkatheter mit der Spitze, dem Ballon und dem ersten Auge durch die Aortenklappe in die Aorta ascendens vorgeschoben wird, bei dem das zweite Auge im Ventrikel verbleibt, bei dem bei lagerichtiger Plazierung des Ballonkatheters der Ballon bis zur vollkommenen Occlusion der Aorta ascendens aufgeblasen wird, bei dem anschließend über das erste Lumen und über das erste Auge aus dem Ballonkatheter austretend eine Kardioplegielösung infundiert wird, bei dem über das zweite Auge und das zweite Lumen Blut in die Herz-Lungenmaschine abgesaugt wird, bei dem unter vorgeschriebener Funktion des Ballonkatheters Koronaranastomosen erstellt werden, bei dem nach der Erstellung der Koronaranastomosen die Occlusion durch den Ballon aufgegeben wird, bei dem anschließend zentrale Anastomosen erstellt werden und bei dem im Anschluß daran der Ballonkatheter aus dem Herzen entfernt wird.

Die Operation beginnt bekannterweise über eine mediane Sternotomie. Ist wegen einer massiven Aortenverkalkung eine normale Kanülierung der Aorta ascendens nicht möglich, so werden diese Patienten femoral über die Leiste arteriell kanüliert. Besteht in der Aorta ascendens ein kalkfreier Bezirk, so kann auch die Aorta ascendens arteriell kanüliert werden. Der venöse Anschluß an die Herz-Lungenmaschine erfolgt in üblicher Technik. Nachdem die Herz-Lungenmaschine vollen Fluß hat und das Herz entlastet ist, wird eine Tabaksbeutelnaht an der Spitze des linken Ventrikels gelegt. Der erfindungsgemäße Ballonkatheter wird nun von außen an das Herz gelegt und die Einlegtiefe des Ballonkatheters anhand der auf dem Katheter angebrachten Graduierung markiert. Innerhalb der Tabaksbeutelnaht wird nun eine Stichinzision ausgeführt und der erfindungsgemäße Ballonkatheter in den linken Ventrikel vorgeschoben. Durch ein weiteres vorsichtiges Vorschieben wird der Katheter durch die Aortenklappe in die Aorta ascendens eingeführt und dies soweit, bis die an dem Ballonkatheter angebrachte Markierung mit der vorher ausgemessenen Länge übereinstimmt. Ist der Ballonkatheter bestimmungsgemäß plaziert, so wird er aufgeblasen, bis die Aorta ascendens vollkommen occludiert ist. Der vollständige Aortenverschluß wird dabei echokardiographisch überwacht. Über das erste Lumen (Kardioplegiekanal) wird die Kardioplegielösung infundiert. Über das zweite Lumen (Ventkanal) wird der linke Ventrikel entlastet und das Blut in die Herz-Lungenmaschine abgesaugt. Die Erstellung der Koronaranastomosen erfolgt in üblicher Technik. Nach der Erstellung der Anastomosen wird die Blockung der Aorta ascendens freigegeben. Das Herz beginnt zu schlagen. Nun werden die zentralen Anastomosen erstellt. Dies kann z.B. als Anschluß an den Truncus brachiocephalicus oder an einem kalkfreien Bezirk der Aorta ascendens erfolgen. Dabei genügt eine einfache Anastomose, weitere Venen werden in die anastomosierte Vene eingepflanzt. Im Anschluß wird der Katheter entfernt und die Tabaksbeutelnaht geknüpft. Diese Stelle wird durch eine Patchnaht gesichert und die Operation beendet. Während der Operation soll eine topische Kühlung des Herzens durch externe Eiswasserspülungen erfolgen.

Wird der erfindungsgemäß weiterentwickelte Ballonkatheter bei Operationen im Rahmen der bisher üblichen minimalinvasiven Koronarchirurgie eingesetzt, so hat dies den Vorteil, daß der erfindungsgemäße Ballonkatheter problemfrei durch eine minimal invasive anterolaterale Thorakotomie über die Herzspitze in das Herz eingeführt werden kann. Die Aorta kann geblockt werden (bei bisherigen minimalinvasiv geführten koronarchirurgischen Eingriffen war dies nicht möglich). Eine Durchführung der Kardioplegie ist unter Einsatz des erfindungsgemäß weiterentwickelten Ballonkatheters möglich und somit das sicherere Nähen von Koronaranastomosen mit hoher Qualität am nicht schlagenden, entlasteten Herzen und ohne Blutungen aus den Herzkranzgefäßen. Bisher erfolgte bei minimalinvasiv geführten Eingriffen das Nähen der Anastomosen am schlagenden Herzen. Unter Einsatz des erfindungsgemäßen Katheters kann nun wie bei den bisher schon etablierten Verfahren das Nähen der Anastomosen im Herzstillstand erfolgen. Über das zweite Lumen (Ventkanal) wird das Herz entlastet. Diese Maßnahme verhindert eine Überdehnung des Herzens und gestattet die Luxation des Herzens.

Zu Beginn der MIC-Operation (minimalinvasive Chirurgie) wird die Herz-Lungenmaschine über die linke bzw. rechte Leiste (Art. femoralis und V. femoralis) angeschlossen. Es erfolgt die bisher in der MIC übliche linksseitige Mini-Thorakotomie. Im Anschluß erfolgt die Präparation der Art. Mammaria sinistra oder dextra. Nach der vollständigen Präparation wird das Ende der Art. mammaria durchtrennt. An der Herzspitze wird eine Tabaksbeutelnaht gelegt. In das Zentrum dieser Tabaksbeutelnaht wird eine Stichinzision gelegt. Über diese Inzision wird der erfindungsgemäß weiterentwickelte Ballonkatheter durch den Ventrikel über die Aortenklappe in die Aorta vorgeschoben. Es erfolgt die Blockung der Aorta ascendens durch den Ballon. Über das erste Lumen des Ballonkatheters (Kardioplegiekanal) wird die Kardioplegielösung in die Aortenwurzel infundiert. Nach dem Herzstillstand wird der linke Ventrikel über das zweite Lumen des Ballonkatheters (Ventkanal) entlastet. Nun kann an dem stillgelegten Herz die Koronaranastomose genäht werden. Nach der Erstellung der Anastomose wird die Aortenblockung freigegeben. Das Herz beginnt zu schlagen. Der Katheter wird entfernt und die Tabaksbeutelnaht geknüpft. Diese Stelle wird noch zusätzlich über eine U-Patchnaht gesichert. Anschließend wird die Operation in üblicher Technik beendet.

Unter Verwendung des erfindungsgemäß weiterentwickelten Ballonkatheters lassen sich mittels der MIC-Technik auch Venenbypässe erstellen. Ist der erfindungsgemäße Ballonkatheter wie beschrieben plaziert, so können am entlasteten Herzen zwischen den Koronargefäßen und den Brustwandarterien sowie zusätzlich mit entnommener V. Saphena Magna vom Patienten Venenbrücken gelegt werden. Nach der schon beschriebenen Erstellung der Koronaranastomosen wird die durch die laterale Thorakotomie gut erreichbare Aorta descendens tangential ausgeklemmt und mit Stanzen werden Löcher in die Arterienwand gestanzt. Die Venen werden an die Aortenwand genäht und anschließend wird die tangentiale Ausklemmung freigegeben. Die Operation wird wie schon beschrieben beendet.

Weitere Vorteile ergeben sich aus der Beschreibung und der beigefügten Zeichnung. Ebenso können die vorstehend genannten und die noch weiter aufgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter.

Die Erfindung ist in der Zeichnung dargestellt und wird anhand von zwei Ausführungsbeispielen näher erläutert.

Es zeigt:
- Fig. 1: einen Abschnitt eines erfindungsgemäßen Ballonkatheters in seinem Spitzenbereich;
- Fig. 2: einen erfindungsgemäßen Ballon in Alleinstellung, wie er an einem Ballonkatheter angebracht ist;
- Fig. 3: einen erfindungsgemäßen Ballonkatheter mit einem transventrikulären Ballon zur Blockung der Aorta mit einem ersten Lumen (Kardioplegiekanal) und einem zweiten Lumen (Ventkanal) zur Anwendung in der Bypasschirurgie.

Die einzelnen Figuren der Zeichnung zeigen den erfindungsgemäßen Gegenstand teilweise stark schematisiert und sind nicht maßstäblich zu verstehen. Die einzelnen gegenständlichen Merkmale in den einzelnen Figuren sind teilweise stark vergrößert dargestellt, damit ihr Aufbau besser gezeigt werden kann.

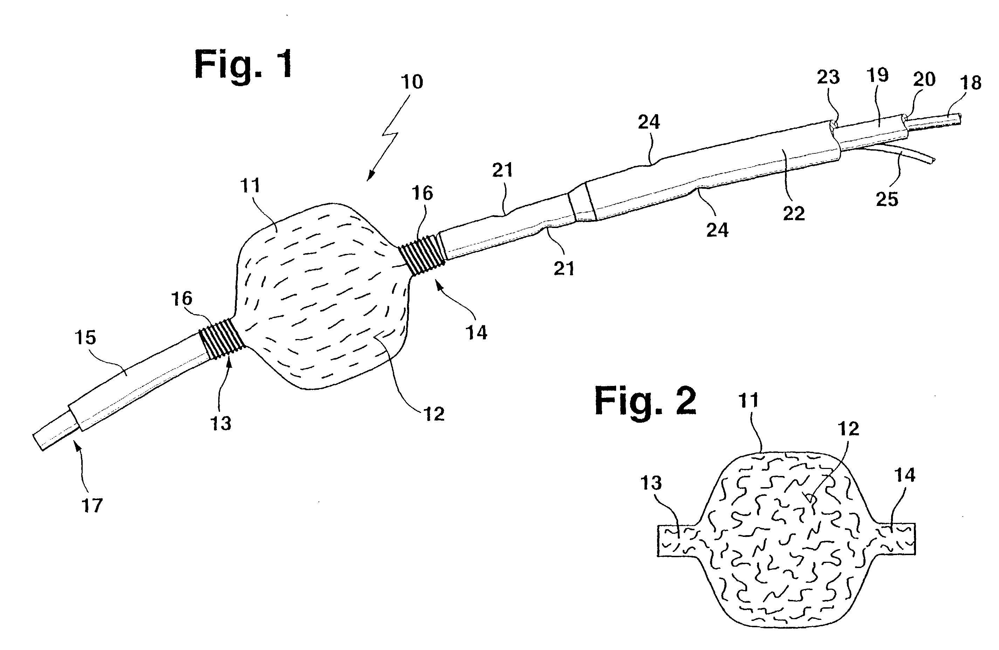

Fig. 1 zeigt mit 10 einen Abschnitt eines Ballonkatheters, der im Spitzenbereich einen Ballon 11 mit einer strukturierten Außenoberfläche 12 aufweist. Die strukturierte Außenoberfläche 12 ist durch Täler und Berge gebildet, die über die axiale Erstreckung des Ballons 11 gesehen einen Durchgang zwischen aneinandergrenzenden Tälern ausschließen. Der gummielastische Ballon 11 ist bevorzugt aus Latex gefertigt und weist eine stabile Grundform sowie einen ersten formstabilen Endbereich 13 und einen zweiten formstabilen Endbereich 14 auf. Die Endbereiche 13, 14 sind in ihrer Dimensionierung an einen Katheterschaft 15 angepaßt, auf den der Ballon 11 aufgezogen wird. In einer bestimmten Position auf dem Katheterschaft 15 wird der Ballon 11 mit dem Katheterschaft 15 verklebt und mit Fäden 16 am Katheterschaft 15 zusätzlich gesichert.

Auf eine Katheterspitze 17 können beliebige anwendungsorientierte Spitzen aufgesetzt und dort dauerhaft befestigt werden.

Der Ballonkatheter 10 weist ein Führungslumen 18 auf, über das ein Führungsdraht in den Ballonkatheter 10 eingeführt werden kann. Über den Führungsdraht kann der Katheterschaft 15 zusätzlich versteift bzw. gelenkt werden.

Der Katheterschaft 15 weist an der patientenabgewandten Seite des Ballons 11 eine erste Katheterummantelung 19 auf, die ein erstes Lumen 20 begrenzt. Das erste Lumen 20 steht mit ersten Augen 21 in Verbindung. Über die ersten Augen 21 ist das erste Lumen 20 mit der Umgebung verbunden. Eine zweite Katheterummantelung 22 ist über der ersten Katheterummantelung 19 angeordnet und begrenzt ein zweites Lumen 23, das mit zweiten Augen 24 in Verbindung steht. Über die zweiten Augen 24 ist das zweite Lumen 23 mit der Umgebung verbunden. Über einen Ballonkanal 25 kann der Ballon 11 mit einer Flüssigkeit, z.B. Kochsalzlösung, aufgeblasen werden. Ebenfalls kann über den Ballonkanal 25 die im Ballon 11 befindliche Flüssigkeit nach außen abgeführt werden.

Fig. 2 zeigt den Ballon 11 der Fig. 1 in Alleinstellung. Der Latex-Ballon 11 wird dadurch hergestellt, daß eine entsprechende Form in eine Latexmilch getaucht wird. Danach wird der auf der Form befindliche Latexfilm in eine Lösung, z.B. Toluol, getaucht, damit der Latex-Ballon 11 nach dem Vulkanisieren eine strukturierte, zerklüftete Außenoberfläche aufweist, die im geblockten Zustand an Gefäßinnenwandungen besonders fluiddicht und schonend haftet. Der Ballon 11 weist neben der strukturierten Außenoberfläche 12 eine bleibende Form auf und ausgeformte Endbereiche 13, 14. Durch die Endbereiche 13, 14 wird der Katheterschaft bis in eine Position hindurchgeschoben, in der er mit dem Ballon 11 verklebt bzw. verbunden wird.

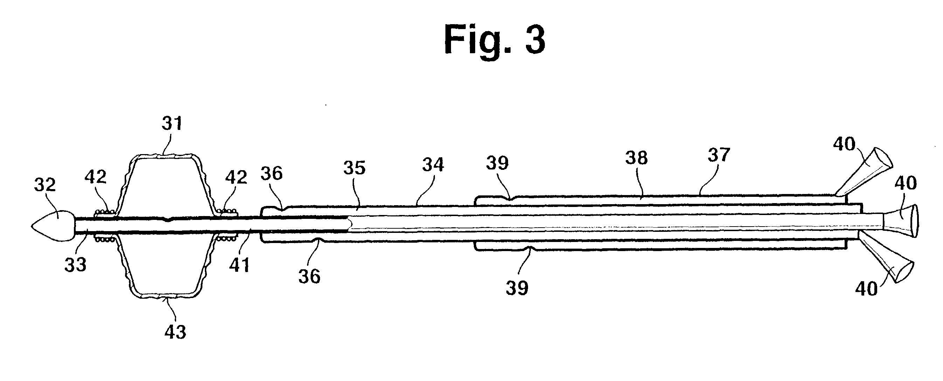

Fig. 3 zeigt ein weiteres Ausführungsbeispiel eines Ballonkatheters 30 mit einem Ballon 31 und einer Spitze 32. Der Ballonkatheter 30 ist weitgehend im Schnitt gezeigt, so daß im Ballonkatheter 30 ausgebildete Lumina gezeigt werden können.

Ein Führungslumen 33 erstreckt sich durch die gesamte axiale Länge des Ballonkatheters 30 und ist in der Spitze 32 verschlossen. In das Führungslumen 33 läßt sich ein Mandrin zur Versteifung und/oder zur Lenkung des Ballonkatheters 30 einführen. Eine erste Katheterummantelung 34 begrenzt ein erstes Lumen 35, das in einem Endbereich erste Augen 36 aufweist. Eine zweite Katheterummantelung 37 begrenzt ein zweites Lumen 38, das in einem Endbereich zweite Augen 39 aufweist. Am patientenabgewandten Ende des Ballonkatheters 30 sind bekannte Konnektiermittel 40 vorgesehen, über die die beschriebenen Lumina mit weiterführenden Leitungen verbunden werden können. Am Katheterschaft 41 sind sowohl die Spitze 32, der Ballon 31 und die erste Katheterummantelung 34 angebracht. Die zweite Katheterummantelung 37 ist über eine gewisse axiale Länge auf der ersten Katheterummantelung 34 ausgebildet. Der Ballon 31 ist mit dem Katheterschaft 41 verklebt und zusätzlich über Fäden 42 am Katheterschaft 41 sicher fixiert. Der Ballon 31 weist eine strukturierte Außenoberfläche 43 auf, mit der er sich unter verstärkter Haftung an angrenzende Oberflächen anschmiegen kann.

Ein Ballonkatheter im medizinischen Anwendungsbereich weist im patientenzugewandten Spitzenbereich einen Ballon 11 auf, der eine strukturierte Außenoberfläche 12 aufweist. Die strukturierte Außenoberfläche 12 ist durch Wellentäler und Wellenberge gebildet, die untereinander keine Verbindung aufweisen. Die strukturierte Außenoberfläche 12 eines Ballons 11 kann sich unter besonderer Haftung an angrenzende Oberflächen anschmiegen. Der Ballonkatheter wird über eine linksventrikuläre Kardiotomie im Spitzenbereich eingelegt und in die Aorta ascendens vorgeschoben. Der Ballon 11 occludiert die Aorta ascendens. Er gestattet die Durchführung der Kardioplegie und die Herzentlastung durch Absaugung.

### Anwendung:

1. bei Verkalkung der Aorta ascendens;
2. bei der Erstellung von LIMA/RIMA-Anastomosen;
3. bei der Erstellung von Venenanastomosen.

## Patentansprüche

1. Ballonkatheter zur Anwendung in der Bypasschirurgie mit einem am Katheterschaft (15; 41) ausgebildeten aufblasbaren Ballon (11; 31) zur Blockung des Lumens der Aorta, wobei ein Führungslumen (18; 33) im Katheterschaft (15; 41) und der Ballon (11; 31) in einem Spitzenbereich des Katheterschaftes (15; 41) ausgebildet sind, mit einem einen Kardioplegiekanal bildenden ersten Lumen (20; 35) zur Infusion einer Kardioplegielösung über mindestens ein erstes Auge (21; 36) und mit einem einen Ventkanal bildenden zweiten Lumen (23; 38) zur Entlastung des Herzens und/oder zum Absaugen von Blut, wobei das Führungslumen (18; 33) am patientenseitigen Ende geschlossen ist und in einer Katheterspitze (17) ausläuft, der Ballon (11; 31) eine strukturierte und/oder zerklüftete Außenoberfläche (12; 43) aufweist, die Lumen (20, 23; 25, 38) durch eine erste Katheterummantelung (19; 34), die am patientenabgewandten Ende des Ballons (11; 31) endet, und eine zweite Katheterummantelung (22; 37) ausgebildet sind, die auf der ersten Katheterummantelung (19; 34) angeordnet und axial beabstandet von dem ersten Auge (21; 36) endet, wobei die zweite Katheterummantelung (22; 37) mindestens ein zweites Auge (24; 39) aufweist.

2. Ballonkatheter nach Anspruch 1, **dadurch gekennzeichnet, dass** der Ballon (11; 31) aus Latex gefertigt ist.

3. Ballonkatheter nach Anspruch 2, **dadurch gekennzeichnet, dass** der Latex-Ballon (11; 31) in einer eigenstabilen Grundform ausgebildet ist, die in einem ersten und einem zweiten Endbereich (13, 14) am Katheterschaft (15; 41) angepasst ist.

4. Ballonkatheter nach einem der Ansprüche 1 bis 3, **dadurch gekennzeichnet, dass** längs des Katheterschaftes (15; 41) eine visuell erkennbare Graduierung angebracht bzw in das Kathetermaterial eingearbeitet ist.

## Claims

1. Balloon catheter for application in bypass surgery, comprising an inflatable balloon (11;31) formed on the catheter shaft (15;41) for blocking the lumen of the aorta, wherein a guide lumen (18;33) is formed in the catheter shaft (15;41) and the balloon (11;31) is formed in the region of a tip of the catheter shaft (15;41), comprising a first lumen (20;35) forming a first cardioplegia channel for infusion of a cardioplegia solution via at least one first eye (21;36) and with a second lumen (23;38) forming a venting channel for relieving the heart and/or for drawing off blood, wherein the guide lumen (18;33) is closed on the patient side end and terminates in a catheter tip (17), the balloon (11;31) has a structured and/or rugged outer surface (12;43), the lumina (20,23;25,38) are formed by a first catheter coating (19;34) which terminates at the end of the balloon (11;31) facing away from the patient, and a second catheter coating (22;37) which is disposed on the first catheter coating (19;34) and terminates at an axial separation from the first eye (21;36), wherein the second catheter coating (22;37) comprises at least a second eye (24;39).

2. Balloon catheter according to claim 1, **characterized in that** the balloon (11; 31) is made from latex.

3. Balloon catheter according to claim 2, **characterized in that** the latex-balloon (11; 31) has an intrinsically stable basic shape adapted at first and second end regions (13, 14) to the catheter shaft (15,41).

4. Balloon catheter according to any one of the claims 1 through 3, **characterized in that** a visually recognizable scale is disposed along the catheter shaft (15; 41) or worked into the catheter material.

## Revendications

1. Cathéter à ballonnet destiné à être utilisé dans la chirurgie de by-pass comportant un ballonnet gonflable (11; 31) formé sur la tige (15; 41) du cathéter et servant à bloquer la lumière de l'aorte, une lumière de guidage (18; 33) étant prévue dans la tige (15; 41) du cathéter et le ballonnet (11; 31) étant formé dans une partie pointue de la tige (15; 41) du cathéter, et comportant une première lumière (20; 35) qui forme un canal de cardioplégie, pour l'injection d'une solution pour cardioplégie, par l'intermédiaire d'au moins un premier oeillet (21; 36) et une seconde lumière (23; 38) formant un canal d'aération et servant à réduire la contrainte appliquée au coeur et/ou à aspirer du sang, et dans lequel la lumière de guidage (18; 33) est fermée sur l'extrémité située du côté du patient et se termine par une pointe de cathéter (17), le ballonnet (11; 31) possède une surface extérieure structurée et/ou fendillée, les lumières (20, 23; 25, 38) sont formées par une première enveloppe (19; 34) du cathéter, qui se termine sur l'extrémité du ballonnet (11, 31) située à l'opposé du patient, et par une seconde enveloppe (22; 37) du cathéter qui est disposée sur la première enveloppe (19; 34) du cathéter et se termine en étant distant axialement du premier oeillet (21; 36), la seconde enveloppe (22; 31) du cathéter possédant au moins un second oeillet (24; 39).

2. Cathéter à ballonnet selon la revendication 1, **caractérisé en ce que** le ballonnet (11; 31) est réalisé en latex.

3. Cathéter à ballonnet selon la revendication 2, **caractérisé en ce que** le ballonnet en latex (11; 31) est agencé avec une forme de basé présentant une stabilité propre, qui est insérée dans des première et seconde parties d'extrémité (13, 14) sur la tige (15; 41) du cathéter.

4. Cathéter à ballonnet selon l'une des revendications 1 à 3, **caractérisé en ce qu'**une graduation, que l'on peut identifier visuellement, est disposée le long de la tige (15; 41) du cathéter ou est formée dans le matériau du cathéter.
